(19) Europäisches Patentamt
European Patent Office
Office européen des brevets

(11) Numéro de publication : **0 542 630 A2**

(12) # DEMANDE DE BREVET EUROPEEN

(21) Numéro de dépôt : **92403057.0**

(51) Int. Cl.$^5$ : **A61K 31/71**

(22) Date de dépôt : **13.11.92**

(30) Priorité : **14.11.91 FR 9113999**

(43) Date de publication de la demande :
**19.05.93 Bulletin 93/20**

(84) Etats contractants désignés :
**AT BE CH DE DK ES FR GB GR IE IT LI LU MC NL PT SE**

(71) Demandeur : **LABORATOIRES MAYOLY SPINDLER S.A.R.L.**
**6, avenue de l'Europe, B.P. 51**
**F-78401 Chatou Cédex (FR)**

(71) Demandeur : **Seman, Michel**
**7, rue La Bruyère**
**F-75009 Paris (FR)**

(72) Inventeur : **Seman, Michel**
**7, rue La Bruyère**
**F-75009 Paris (FR)**

(74) Mandataire : **Orès, Bernard et al**
**Cabinet ORES 6, Avenue de Messine**
**F-75008 Paris (FR)**

(54) **Utilisation de dérivés d'amphotéricine B, comme inhibiteurs de protéases.**

(57) Nouvelle utilisation de l'amphothéricine B, ou d'un dérivé de celle-ci, comme inhibiteur de protéases.
Application à l'obtention de médicaments inhibiteurs de la multiplication d'agents pathogènes, en particulier le VIH.

EP 0 542 630 A2

La présente invention est relative à l'utilisation de dérivés de macrolides polyéniques et en particulier d'amphotéricine B, comme inhibiteurs de protéases, et aux applications desdits inhibiteurs pour l'obtention de médicaments, destinés en particulier au traitement de l'infection par le virus de l'immunodéficience humaine (VIH).

Depuis l'identification des virus VIH-1 et VIH-2 comme agents du SIDA, de nombreuses recherches ont été faites dans le but de comprendre le mécanisme de l'infection virale, et d'obtenir des traitements antiviraux efficaces.

Jusqu'à présent, des analogues de nucléosides tels que l'AZT (3'-azido-2',3'-didéoxythymidine) et le ddI (2',3'-didéoxyinosine) ont été utilisés pour le traitement du SIDA et ont abouti à une amélioration de l'état clinique des patients. Malheureusement, des effets secondaires sévères du traitement par l'AZT, en particulier une dégénérescence de la moelle osseuse, ont été rapportés, et en outre, de nombreux patients tolèrent mal la drogue ou deviennent réfractaires à ses effets bénéfiques.

En ce qui concerne le ddI, son utilisation n'est encore qu'au stade expérimental, et son efficacité demande une évaluation complémentaire.

Il a, par ailleurs, été montré que l'Amphotéricine B (AmB) et certains de ses dérivés sont actifs *in vitro* contre plusieurs virus à ADN et ARN à enveloppe lipidique, et contre différentes souches d'herpes virus. Le méthylester de l'Amphotéricine B (AME) [SCHAFFNER et al., Biochem. Pharm., 35, 4110-4113, 1986], ainsi que l'Amphotéricine B encapsulée dans les liposomes [PONTANI et al., Antiviral. Res. 11, 119-126 (1989)] ont également été décrits comme inhibiteurs de la mort cellulaire et de l'expression virale dans des cellules T infectées *in vitro* par VIH-1. Comme il est connu d'autre part que l'amphotéricine B et ses dérivés interagissent avec le cholestérol des membranes cellulaires, ce qui provoque une fluidification de ces dernières, il a été supposé que ce mécanisme était responsable de leur activité antivirale, soit par destruction du virus, soit par prévention de sa pénétration dans les cellules cibles. HANSEN et al. [Antiviral Research, 14, 149-160, (1990)] ont étudié l'activité *in vitro* de différents dérivés d'anphothéricine sur le virus VIH-1. D'après les résultats de cette étude, l'AME agirait en tant que cytoprotecteur, en inhibant l'infection cellulaire par le VIH libre : l'incubation de lymphocytes sains en présence d'AME préalablement à l'infection *in vitro* par un isolat de VIH inhibe l'infection ; en revanche, lorsque des lymphocytes infectés par VIH sont préincubés en présence d'AME, leur capacité à fusionner avec des cellules non-infectées pour former des syncytia est augmentée. Un autre des dérivés étudiés, le MCG (méthyl ester de N-(N'-(2-4'-méthylmorpholino)N''-éthyl guanyl) amphothéricine B), ne possède, selon cette étude, pas de propriétés cytoprotectrices, mais aurait une action antivirale directe : dans le cas du MCG, c'est la préincubation avec le virus, et non avec les cellules qui inhibe l'infection virale.

L'amphotéricine B est actuellement utilisée chez les patients atteints de SIDA, dans le traitement des infections à germes opportunistes, en particulier des mycoses profondes qui surviennent fréquemment chez ces malades. Des études cliniques ont montré qu'une thérapie antifongique doit être maintenue de façon indéfinie afin de prévenir les rechutes, ou même utilisée de manière préventive. Cependant, des effets secondaires fréquents telles que la fièvre ou une néphrotoxicité grave sont associés au traitement à long terme par l'amphotéricine B.

En recherchant des antifongiques plus actifs, bénéficiant d'une toxicité réduite et d'une efficacité améliorée, l'équipe des Inventeurs a récemment obtenu en substituant la fonction mycosamine de l'amphothéricine B par un groupe amino-1-désoxy-1-cétose qui peut lui-même être substitué, une série de nouveaux dérivés plus solubles et moins toxiques que l'amphothéricine B ; ces dérivés, et leur utilisation comme antifongiques, font l'objet de la Demande de Brevet européen n°428 440.

Or, les Inventeurs ont maintenant testé l'action de certains de ces dérivés, de formule générale (I), ci-dessous,

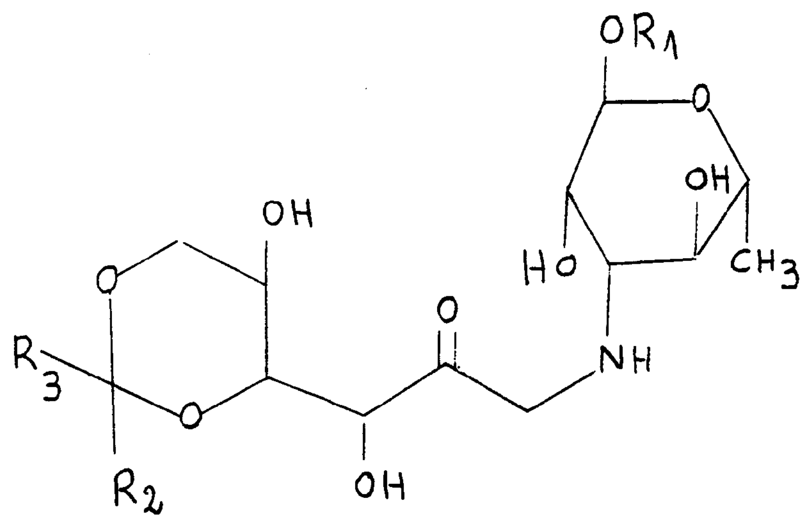

(I)

dans laquelle :

- R1 représente la partie macrocyclique du macrolide polyénique,
- R2 représente un atome d'hydrogène ou un groupe méthyle,
- et R3 représente un groupe méthyle, éthyle, gropyle, phényle,

comme inhibiteurs de l'infection par VIH.

Ils ont ainsi constaté que ces dérivés inhibent les effets cytopathogènes induits par VIH-1, VIH-2, et SIV (virus de l'immunodéficience du singe) et inhibent presque complètement la réplication de VIH dans tous les types cellulaires testés, à des concentrations très inférieures aux concentrations cytotoxiques.

Des expériences réalisées par les Inventeurs en vue d'élucider le mécanisme d'action des dérivés de formule générale (I) ont montré qu'ils sont inactifs sur la transcriptase inverse *in vitro*, et actifs sur des mutants de VIH AZT-résistants.

L'activité anti-VIH de ces dérivés présente en outre des particularités inattendues au vu des mécanismes d'action décrits dans l'art antérieur pour d'autres dérivés d'amphothéricine B.

En particulier les Inventeurs ont observé que, contrairement à ce qui a été décrit dans le cas de l'AME (HANSEN et al., publication précitée), ni le prétraitement des cellules par les dérivés d'AME de formule générale (I) avant l'infection, ni le prétraitement du virus lui-même n'ont d'effet inhibiteur sur la réplication virale.

D'autre part, les Inventeurs ont observé que les dérivés de formule générale (I) inhibent les effets cytopathologiques du virus de la rhinite humaine (HRV14) dans des cultures de cellules Héla-Ohio ; or, contrairement au virus VIH-1, le virus HRV14 n'est pas un virus enveloppé.

Les Inventeurs ont donc émis l'hypothèse qu'un mécanisme commun, différent de l'interaction avec le cholestérol des membranes cellulaires et/ou de l'enveloppe virale serait impliqué dans les activités anti-VIH et anti-rhinovirus des dérivés de formule générale (I).

Les Inventeurs ont d'autre part observé que les dérivés de formule générale (I) constituaient des inhibiteurs de protéases.

Or, la multiplication de HIV-1, comme celle du rhinovirus, nécessitent l'intervention d'enzymes protéolytiques : dans le cas du rhinovirus, deux protéases participent à la maturation de la polyprotéine issue de la traduction de l'ARN viral ; dans le cas de HIV-1, au moins une protéase cellulaire contribuerait à la fusion entre l'enveloppe virale et la membrane cellulaire, ainsi que dans la formation de syncytia, et une protéase virale (protéase p10), participerait à la maturation des protéines virales.

Les dérivés de formule générale (I) sont inactifs *in vitro* sur la protéase p10, qui est une aspartyl-protéase ; en revanche, ils sont actifs sur la trypsine, et sur d'autres sérine-protéases.

Les Inventeurs ont recherché si d'autres dérivés de l'amphothéricine B possédaient la même activité inhibitrice des protéases ; ils ont observé que des dérivés dans lesquels la fonction mycosamine est substituée par un groupe amino-1-désoxy-1-cétose, tels que ceux décrits dans la Demande de Brevet européen n°428 440, ont une activité similaire à celle des dérivés de formule (I) ; en outre, l'amphothéricine B, ainsi que tous les dérivés de celle-ci testés par les Inventeurs, possèdent, bien qu'à des degrés très divers, une certaine activité inhibitrice sur la trypsine ; l'importance de cette activité semble liée à la présence d'un substituant sur la mycosamine.

Les nouvelles propriétés des dérivés d'amphothéricine B mises en évidence par les inventeurs, et en particulier les propriétés d'inhibiteurs des sérine-protéases, permettent de proposer de nouvelles utilisations de

ces dérivés. En particulier, ceux de ces dérivés dont l'activité inhibitrice des sérine-protéases est significative sont utilisables pour le traitement de maladies impliquant un agent pathogène dont le cycle de multiplication fait intervenir au moins une enzyme protéolytique, du type sérine protéase.

La présente invention a pour objet l'utilisation de l'amphothéricine B, ou d'un dérivé de celle-ci, pour l'obtention d'un inhibiteur de protéases, actif en particulier sur des sérine-protéases, telles que la trypsine, la kallicréine, l'élastase leucocytaire.

Selon un mode de réalisation préféré de la présente invention, le dérivé d'amphothéricine B utilisé est choisi parmi les dérivés dont l'activité inhibitrice sur la trypsine est au moins deux fois supérieure à celle de l'amphothéricine B.

Selon un autre mode de réalisation préféré de la présente invention, la fonction mycosamine du dérivé d'amphothéricine B utilisé est substituée.

Selon une disposition préférée de ce mode de réalisation, la fonction mycosamine est substituée par un groupe aspartyle.

Selon une autre disposition préférée de ce mode de réalisation, la fonction mycosamine est substituée par un groupe amino-1-désoxy-1-cétose, lui même éventuellement substitué.

Selon une modalité préférée de cette disposition, le dérivé utilisé répond à la formule générale (I),

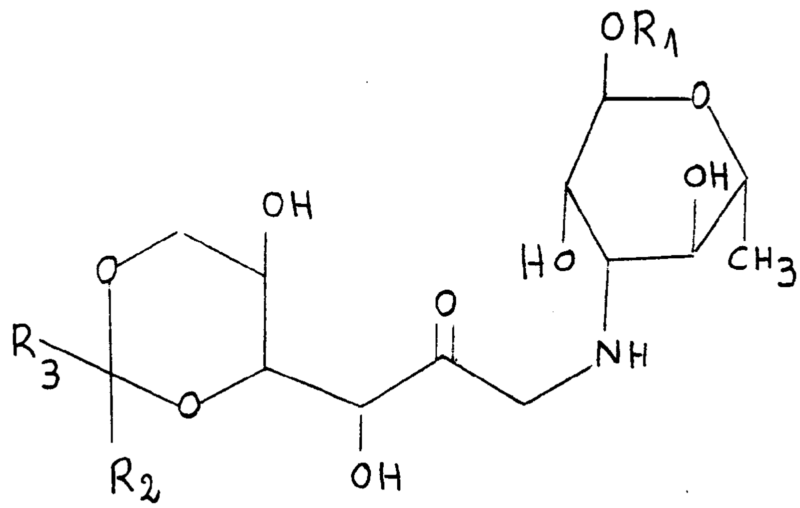

(I)

dans laquelle :

- R1 représente la partie macrocyclique d'un macrolide polyénique, de préférence l'amphotéricine B,
- R2 représente un atome d'hydrogène ou un groupe méthyle,
- et R3 représente un groupe méthyle, éthyle, propyle, phényle.

La présente invention a également pour objet l'utilisation des inhibiteurs de sérine-protéases tels que définis ci-dessus pour l'obtention de produits, en particulier de médicaments, inhibant la multiplication d'un agent pathogène dont le cycle de multiplication implique l'intervention d'au moins une enzyme protéolytique, du type sérine-protéase.

Les dérivés d'amphothéricine B préférés pour cette utilisation sont ceux dont l'activité inhibitrice sur la trypsine est au moins deux fois supérieure à celle de l'amphothéricine B.

Selon un mode de réalisation préféré de la présente invention, ledit agent pathogène est un virus.

Selon une disposition préférée de ce mode de réalisation, ledit virus est le virus VIH-1.

Selon une autre disposition préférée de ce mode de réalisation, ledit virus est le virus VIH-2.

Selon encore une disposition préférée de ce mode de réalisation, ledit virus est le virus SIV.

Selon encore une autre disposition préférée de ce mode de réalisation, ledit virus est un rhinovirus, en particulier le virus de la rhinite humaine.

Selon un autre mode de réalisation préféré de la présente invention, ledit agent pathogène est une bactérie.

Selon encore un mode de réalisation préféré de la présente invention, ledit agent pathogène est un organisme eucaryote, parasite de l'homme ou des animaux.

La présente invention sera mieux comprise à l'aide du complément de description qui va suivre, se réfère à des exemples détaillés concernant l'évaluation de l'effet inhibiteur des protéases de différents dérivés d'amphothéricine B, et de l'activité sur VIH, SIV et HRV14 d'un dérivé inhibiteur de la trypsine, le 1-déoxy-1-amino-4,6-O-benzylidène-D-fructosyl-AmB (dénommé dans ce qui suit DABF-AmB).

Il va de soi toutefois que ces exemples sont donnés uniquement à titre d'illustration de l'objet de l'Invention, dont ils ne constituent en aucune manière une limitation.

## I) DETERMINATION DE L'ACTIVITE INHIBITRICE DE DIFFERENTS DERIVES D'AMPHOTHERICINE SUR DES SERINE-PROTEASES

1) Inhibition de la trypsine :

Les différents dérivés testés (AmB, AmB méthyl ester (AME), N-acétyl-AmB, N-aspartyl-AmB, N-acétyl-AME, DABF-AmB) ont été ajoutés, à la concentration de 1 μM à une solution de trypsine $3{,}6.10^{-9}$ M dans un tampon Tris-HCl 0,1M pH 8.2, en présence de DTNB 0,35mM ; la réaction est déclenchée par addition de la quantité de substrat (Z-lysinethiobenzylester) nécessaire pour une concentration finale de $0{,}6.10^{-6}$ M. La réaction est suivie par mesure de la densité optique à 412nm.

Les résultats sont représentés à la figure 1 ; ils sont exprimés en % d'inhibition par rapport à un témoin sans inhibiteur. Dans les conditions de l'expérimentation, le DABF-AmB inhibe à plus de 60% l'activité de la trypsine ; les autres dérivés testés ont une certaine activité (d'environ 10% d'inhibition pour l'AmB et l'AME jusqu'à environ 40% d'inhibition pour le N-aspartyl-AmB), mais aucun ne possède une activité comparable à celle du DABF-AmB. D'autre part, il a été vérifié que le benzylidène glucoside seul n'a pas d'action inhibitrice.

Le tableau I illustre l'effet inhibiteur du DABF-AmB sur la trypsine à différentes concentrations. L'inhibition est totale à une concentration de $10^{-4}$M.

TABLEAU I

% D'INHIBITION DE LA TRYPSINE PAR DABF-AmB ET AMB

| | $10^{-7}$M | $10^{-6}$M | $10^{-5}$M | $10^{-4}$M |
|---|---|---|---|---|
| DABF-AmB | 20 | 50 | 95 | 100 |
| AMB | 5 | 10 | 30 | NT* |

* Non testé

Ces résultats suggèrent que la fonction impliquée dans l'effet inhibiteur des protéases est présente sur la molécule d'AmB mais qu'elle n'est pas accessible tant que la modification de solubilité et de comportement en solution n'a pas été introduite. La présence d'un substituant sur le NH2 de la mycosamine semble augmenter l'activité inhibitrice de la molécule.

2) Activité du DABF-AmB sur différentes protéases

Le DABF-AmB a été testé à la concentration de 1 μM, sur différentes protéases (papaïne, chymotrypsine, kallicréine, élastase leucocytaire, cathepsine D, protéase p10 recombinante) dans les conditions et en présence des substrats appropriés à chacune des protéases testées.

Les résultats sont représentés à la figure 2, en pourcentage de l'inhibition observée sur la trypsine dans les mêmes conditions. Le DABF-AmB inhibe la kallicréine (résultats non montrés) et l'élastase leucocytaire qui sont, comme la trypsine, des sérines-protéases mais en revanche, n'inhibe pas la chymotrypsine. Le DABF-AmB n'inhibe pas la papaïne (cystéine-protéase) ni la cathepsine D et la protéase p10 (aspartyl-protéases).

Une composante essentielle de l'activité du DABF-AmB sur les virus VIH et SIV résulte certainement de son action sur des sérine-protéases de la membrane cellulaire, qui interviennent dans l'infection virale.

La mesure de l'activité inhibitrice de dérivés d'amphotéricine B sur une sérine-protéase telle que la trypsine, peut constituer un criblage, en vue de permettre la sélection des dérivés les plus actifs sur le VIH.

## II) - ACTION DU DABF-AmB SUR LES VIRUS VIH ET SIV

### PROTOCOLES GENERAUX

Le sel de N-méthyl glucamine du DABF-AmB est préparé comme décrit dans la Demande Européenne n° 428 440. Il est dissous dans une solution stérile de glucose à 5% dans l'eau, puis dilué dans du milieu de culture.

Les cellules T (Jurkat et CEM) utilisées sont très permissives à l'infection par le VIH. Elles sont cultivées dans du milieu RPMI 1640 complémenté avec 10% de sérum de veau foetal, 2 mM de glutamine et un mélange de pénicilline et streptomycine. Les cellules mononucléaires du sang périphérique, sont obtenues à partir du sang de donneurs sains, isolées sur un gradient de Ficoll Hypaque et stimulées avec 10 μg/ml de Phytohémagglutinine A (PHA), pendant deux jours, dans du milieu RPMI 1640 auquel sont ajoutés préalablement à l'infection par VIH, de l'amikacine et de la vancomycine (10 mg/ml), du polybrène (1 mg/ml) et du sérum anti-interféron-gamma (5 ( U/ml). Après infection, les PMBCs sont cultivés ($10^5$ par puits) dans des plaques de 96 puits à fond plat dans le même milieu RPMI 1640 (200 μl par puits).

Des préparations infectieuses de la souche LAV/Bru de VIH-1 sont obtenues à partir de surnageants de cultures de cellules CEM (clone 13) infectées de façon chronique, filtrés à travers un filtre de 0,45 μm.

Pour l'infection, des cellules fraîches de type Jurkat ou CEM sont centrifugées et le culot est incubé pendant 2 heures à 37°C avec la préparation virale (10000 cpm d'activité transcriptase inverse/$10^6$ cellules, ce qui correspond à une multiplicité d'infection de 0,04 à 0,1). Les cellules sont alors lavées et resuspendues (0,5 x $10^6$/ml) dans du milieu de culture frais. L'activité transcriptase inverse est déterminée à des temps précis après l'infection, à partir du surnageant acellulaire des cellules infectées.

Les cellules PBMCs (2 x $10^6$/ml) sont infectées avec les mêmes virus et dans les mêmes conditions que les cellules Jurkat ou CEM. Elles sont ensuite lavées et resuspendues dans du milieu de culture frais, dans des plaques de 96 puits à fond plat ($10^5$ cellules par puits dans 200 ml).

L'activité transcriptase inverse est déterminée aux temps indiqués, à partir du surnageant de culture, selon le protocole décrit par REY et al., Biochem. Res. Comm. 121, 126-133 (1984).

L'antigène viral p24 a été quantifié, dans le surnageant de cellules infectées, par essai radioimmunologique, en utilisant le protocole préconisé par le fabricant (ABBOTT, Division Diagnostic).

L'expression membranaire du CD4 et de la Gp120 est mesurée à l'aide respectivement des anticorps monoclonaux OKT4 (0,5 μg/ml), 110-4 (2 μg/ml), révélés par un anticorps de chèvre anti-Ig de souris conjugué à l'isothiocyanate de fluorescéine.

La fluorescence est mesurée à l'aide d'un compteur de cellules FACSTAR (BECTON DICKINSON, MOUNTAIN VIEW, CA). Les résultats sont exprimés en intensité moyenne de fluorescence.

L'anticorps monoclonal OKT4 spécifique de CD4 a été acheté auprès de ORTHO PHARMACEUTICAL CORPORATION, (Raritan, NJ).

L'anticorps monoclonal 110.4 (GENETIC SYSTEMS CORP., Seattle, WA), dirigé contre la gp110 reconnait également la gp120 à la surface des cellules T.

### EXEMPLE 1. - Action sur l'activité transcriptase inverse

Les cellules T de type Jurkat et CEM sont infectées avec la souche de VIH-1 LAV/BRU.

L'infection virale et la réplication sont suivies par mesure de l'activité transcriptase inverse dans les surnageants de cultures.

Comme le montre la Figure 3, l'activité transcriptase inverse est décelable à partir du 4ème jour d'infection pour les deux types cellulaires et un pic important d'activité, à 3,2 x $10^6$ et 1,5 x $10^6$ cpm/ml, est observé au 7ème et 8ème jour après l'infection, respectivement dans les cellules Jurkat (Figure 3a) et CEM (Figure 3c). L'activité transcriptase inverse décroît alors jusqu'à un plateau (environ 0,2-0,4 x $10^6$ cpm/ml). Ce plateau reste stable pendant plus de 2 mois, et reflète une infection chronique des cellules T. Les cellules infectées demeurent viables, et dans les deux mois qui suivent l'infection on n'observe ni mort cellulaire, ni inhibition de la croissance cellulaire significatives.

Pour mesurer l'activité anti-VIH du DABF-AmB, des cellules infectées sont cultivées en présence de doses croissantes (entre 0,1 μM et 100 μM) du produit ; celui-ci est ajouté juste après l'inoculation, et les concentrations sont maintenues constantes pendant l'infection, en diluant de moitié, tous les 3 jours, les cellules en croissance dans du milieu frais contenant la concentration souhaitée.

Les résultats correspondants sont illustrés par les figures 3b et 3d : en ordonnée est représentée l'activité transcriptase inverse, mesurée au 8ème jour de l'infection virale, et en abscisse la concentration en DABF-AmB.

L'inhibition de l'activité transcriptase inverse est observée aussi bien dans les cellules Jurkat (Figure 3b)

que dans les cellules CEM (Figure 3d), à partir de 5 µM et l'activité est complètement inhibée (inhibition de 88%, N = 4) à 10 µM de DABF-AmB. Cette dernière concentration a donc été choisie pour la suite des études sur l'activité anti-VIH du produit. Aucune inhibition significative n'est observée pour des concentrations de DABF-AmB inférieures à 1 µM.

La viabilité cellulaire a été estimée en parallèle. A des concentrations supérieures à 100 µM, aucun effet cytotoxique du DABF-AmB n'a été observé, ni sur les cellules Jurkat ni sur les cellules CEM. Il apparaît donc que le DABF-AmB inhibe la réplication de VIH, à des doses dépourvues de cytotoxicité.

Il a été vérifié que le DABF-AmB n'a aucune action directe sur l'activité transcriptase inverse, et que, en conséquence, la décroissance de cette activité ne peut être due à une inhibition directe de l'enzyme par le DABF-AmB.

EXEMPLE 2 - Action sur l'expression de l'antigène viral soluble p24

L'expression de l'antigène viral soluble p24 a été recherchée parallèlement à l'activité transcriptase inverse, dans les surnageants de cultures cellulaires.

Les cellules Jurkat, ainsi que les cellules CEM non traitées produisent de grandes quantités de p24 au 8ème jour après l'infection.

De même que pour l'activité transcriptase inverse, l'addition continue de DABF-AmB à une concentration de 10 µM, maintenue constante pendant 12 jours après l'infection, diminue fortement la quantité d'antigène p24 (88% d'inhibition, N = 3) dans les deux lignées cellulaires.

Ces résultats sont illustrés dans la figure 4 qui représente la production d'antigène p24, 4 jours et 8 jours après l'infection :

■ Cellules infectées non traitées

Cellules traitées par le DABF-AmB

Cellules traitées par l'AmB(10 µM).

Le traitement par l'AmB est effectué dans les mêmes conditions que le traitement par le DABF-AmB. Ces résultats montrent que l'efficacité du DABF-AmB sur la multiplication du VIH est très supérieure à celle de l'AmB.

Une inhibition significative est observée pour les concentrations en DABF-AmB supérieures à 5µM.

Dans une autre expérience, 10 µM de DABF-AmB sont ajoutés, ou bien en prétraitement 24 heures avant l'infection, ou bien pendant les 2 heures de l'étape d'inoculation, ou bien en traitement continu, comme décrit précédemment, pendant les 12 jours suivant l'infection.

Dans ce cas, l'addition de DABF-AmB préalablement à l'infection, ou bien pendant l'étape de 2 heures qui correspond à l'adsorption virale, n'entraine aucune inhibition significative de la sécrétion de p24, ou de l'activité transcriptase inverse.

EXEMPLE 3 - Action sur l'expression de l'antigène viral gp120 et l'antigène cellulaire CD4

La réplication de VIH-1 peut également être suivie par l'expression de la glycoprotéine d'enveloppe gp120 à la surface des cellules infectées, ainsi que par la diminution de l'antigène de membrane CD4.

Des analyses d'immunofluorescence sont effectuées au 8ème jour de l'infection afin de vérifier si l'inhibition de la réplication de VIH par le DABF-AmB a pour résultat une expression membranaire réduite des antigènes viraux.

Au 8ème jour d'infection dans les cellules Jurkat et CEM non traitées, on observe des niveaux élevés de gp120 et des niveaux réduits de CD4 (ce qui est vérifié par l'utilisation des anticorps monoclonaux 110.4 et OKT4 respectivement).

Un traitement continu avec 10 µM de DABF-AmB diminue considérablement l'expression membranaire de gp120 dans les deux lignées cellulaires. Parallèlement, le niveau de l'antigène de membrane CD4 dans les cellules infectées traitées au DABF-AmB augmente jusqu'à atteindre celui des cellules non infectées.

Le niveau de l'antigène de membrane CD3 est inchangé dans les cellules infectées traitées ou non traitées.

EXEMPLE 4 - Action du DABF-AmB sur la réplication du VIH dans les PBMC

Des PBMcs isolés d'individus sains, sont infectés par VIH-1. La production d'antigène p24, ainsi que l'activité transcriptase inverse peuvent être détectées dans les surnageants des cultures infectées, 3 jours après l'infection. Le maximum d'activité transcriptase inverse et de synthèse de p24 sont observées respectivement

entre le 6ème et 7ème jour, et le 5ème et le 6ème jour. Aucune mortalité cellulaire n'est observée à ce moment-là.

Les PBMC infectés sont traités de façon continue comme décrit précédemment pour des cellules Jurkat et CEM, par des concentrations croissantes de DABF-AmB.

L'effet du DABF-AmB sur l'activité transcriptase inverse et sur la sécrétion de P24 est évalué au 6ème jour.

comme dans le cas des cellules Jurkat et CEM, le produit inhibe presque totalement l'activité transcriptase inverse, ainsi que la synthèse de P24 (92±,4 et 88±,6% d'inhibition respectivement, N = 3) à une concentration de 10 μM.

Des concentrations inférieures à 1 μM ne produisent qu'une faible inhibition, tandis que des concentrations supérieures à 20 μM n'ont pas pour effet une augmentation significative de l'action anti VIH.

D'autre part, le DABF-AmB est dépourvu d'effet cytotoxique sur les PBMc à des concentrations allant jusqu'à $10^{-3}$ M.

Il apparaît donc que le DABF-AmB inhibe de façon efficace la réplication de VIH-1 dans des cellules T humaines normales, à des concentrations non cytotoxiques.

EXEMPLE 5 - Protection par DABF-AmB des cellules CD4 dans les cultures de thymocytes humains infectés par le VIH

Des thymus humains ont été obtenus au cours d'opérations chirurgicales cardiaques correctives chez des enfants âgés de 3 à 7 ans. Les tissus ont été coupés en fragments et les globules rouges ont été éliminés par traitement avec un tampon Tris $NH_4Cl_2$. Après lavage dans du tampon PBS, des suspensions cellulaires ont été réalisées dans un milieu RPMI 1640 contenant 10°% de sérum humain type AD. Les cellules mortes ont été éliminées par passage sur une colonne stérile de laine de nylon.

Les cellules ont été infectées par le VIH LAV/BRU comme indiqué ci-dessus dans les protocoles généraux, puis remises en culture en présence de PHA, d'anticorps anti-CD2 ou anti-CD3 et d'IL2 et cultivées pendant 10 jours à raison de 1 x $10^7$ cellules/ml. Après 10 jours, la production virale a été évaluée par la mesure de l'activité RT dans les surnageants et la distribution CD4/CD8 a été évaluée par cytométrie de flux en double fluorescence au moyen d'anticorps anti CD4 (OKT4) marqués au FITC et anti-CD8 (OKT8), marqués à la phycoérythrine. DABF-AMB a été ajouté dans les cultures immédiatement après infection et maintenu pendant toute la durée de la culture.

Le DABF -AmB ne modifie pas significativement, même à 10 μM, la distribution des cellules CD4 et CD8, dans des cultures de thymocytes non infectés activés par la PHA, par des anticorps anti-CD2 ou des anti - CD3. Tout juste observe-t-on une légère augmentation de la population CD4+ CD8+ .

Dans les cultures infectées et non traitées, on observe une forte diminution de la population CD4+CD8+ et de la population CD4+CD8- en correspondance avec une forte production virale. En présence de DABF-AmB, on observe le maintien ou la réapparition des cellules CD4+ dans les cultures infectées, proportionnellement à la concentration du produit. Cette réapparition, ou ce maintien des cellules CD4+ sont corrélés avec une diminution de l'activité RT dans les surnageants.

Le DABF-AmB inhibe donc l'infection des thymocytes CD4+ par le VIH et restaure une distribution normale des sous populations cellulaires thymiques dans les cultures infectées.

EXEMPLE 6 - Action du DABF-AmB sur les phases précoces de l'infection par le VIH

Des cellules HeLa transfectées par le gène CD4 et infectables par le VIH, porteuses du gène bactérien *LacZ* placé sous le contrôle du LTR du VIH [ROCANCOURT et al., J. Virol., 64:2660-2668, (1990)] ont été infectées par le VIH-1 BRU. 24 heures après l'infection de cultures monocouches de cellules H12 dans des puits de 1 ml, à différentes doses virales et en présence de différentes concentrations de DABF-AmB, la transactivation du gène LTR par le gène Tat du virus a été révélée par la recherche de colonies bleues résultant de l'activation de la β-galactosidase du gène *Lac-Z*. Cette détection a été réalisée après fixation des cellules par une incubation d'une heure en présence de 5-bromo-4-chloro-3-indoyl-β-D-galactopyranoside, substrat de l'enzyme. A la concentration de 10 μM, DABF-AmB inhibe complètement l'apparition de colonies bleues indiquant que le produit agit précocément dans le cycle d'infection.

EXEMPLE 7 - Inhibition par DABF-AmB des effets cytopathogènes induits dans les cellules HUT78 infectées par les virus SIVmac251 et HIV-2-nod.

Des cellules HUT78 ont été mises en culture dans des plaques de 96 puits à raison de 2 x $10^4$ cellules par

puits dans du milieu RPM1640. Elles ont été infectées par différentes concentrations virales et du DABF-AmB a été ajouté immédiatement après l'infection, à différentes concentrations. La formation de syncitia a été évaluée à 3 et 5 jours par lecture directe au microscope inversé (test semi-quantitatif).

Comme l'indiquent les tableaux II (infection par VIH-2) et III (infection par SIV), DABF-AmB inhibe (-) de façon dose dépendante et en fonction de la charge virale la formation de syncitia dans les cultures (+). Il est donc actif sur les effets cytopathogènes induits par HIV2 et par SIV.

TABLEAU II

EFFETS ANTIVIRAUX DU COMPOSE DABF-AmB EVALUES SUR LIGNEE CELLULAIRE HUT78 INFECTEE PAR HIV2-rod.

| | Témoins | | | | 10 TCID50 | | | | 100 TCID50 | | | |
|---|---|---|---|---|---|---|---|---|---|---|---|---|
| DABF-AmB/HUT78/HIV2 | 1 | 2 | 3 | 4 | 5 | 6 | 7 | 8 | 9 | 10 | 11 | 12 |
| tem nég./pos. | - | - | - | - | ++ | + | + | ++ | ++ | +++ | ++ | +++ |
| 100 µM | - | - | - | - | forte cytotoxicité | | | | - | - | - | - |
| 50 µM | - | - | - | - | faible cytotoxicité | | | | - | - | - | - |
| 10 µM | - | - | - | - | - | - | - | - | + | + | - | - |
| 5 µM | - | - | - | - | - | + | - | + | + | + | + | + |
| 1 µM | - | - | - | - | ++ | + | + | ++ | +++ | ++ | ++ | +++ |
| 0,5 µM | - | - | - | - | ++ | ++ | + | + | +++ | ++ | ++ | +++ |
| 0,1 µM | - | - | - | - | + | + | ++ | + | +++ | ++ | +++ | +++ |

**Lecture à 3 jours**

| | Témoins | | | | 10 TCID50 | | | | 100 TCID50 | | | |
|---|---|---|---|---|---|---|---|---|---|---|---|---|
| DABF-AmB/HUT78/HIV2 | 1 | 2 | 3 | 4 | 5 | 6 | 7 | 8 | 9 | 10 | 11 | 12 |
| tem nég./pos. | - | - | - | - | ++ | + | ++ | ++ | +++ | +++ | +++ | +++ |
| 100 µM | - | - | - | - | cytotoxicité | | | | - | - | - | - |
| 50 µM | - | - | - | - | - | - | - | - | - | - | - | - |
| 10 µM | - | - | - | - | - | - | - | - | + | ++ | - | - |
| 5 µM | - | - | - | - | - | + | - | + | ++ | ++ | + | + |
| 1 µM | - | - | - | - | +++ | +++ | ++ | ++ | +++ | +++ | +++ | +++ |
| 0,5 µM | - | - | - | - | ++ | ++ | ++ | ++ | +++ | +++ | +++ | +++ |
| 0,1 µM | - | - | - | - | + | + | ++ | ++ | +++ | +++ | +++ | +++ |

**Lecture à 5 jours**

TABLEAU III

EFFETS ANTIVIRAUX DU COMPOSE DABF-AmB EVALUES SUR LIGNEE CELLULAIRE HUT78 INFECTEE PAR SIVmac251.

| | Témoins | | | 10 TCID50 | | | 100 TCID50 | | | 500 TCID50 | | |
|---|---|---|---|---|---|---|---|---|---|---|---|---|
| DABF-AmB'/HUT78/SIV | 1 | 2 | 3 | 4 | 5 | 6 | 7 | 8 | 9 | 10 | 11 | 12 |
| tem nég./pos. | - | - | - | - | - | - | + | + | + | ++ | ++ | + |
| 100 µM | - | - | - | forte cytotoxicité | | | | | - | - | - | - |
| 50 µM | - | - | - | - | faible cytotoxicité | | | | - | - | - | - |
| 10 µM | - | - | - | - | - | - | - | - | - | - | - | - |
| 5 µM | - | - | - | - | - | - | - | - | + | - | + | - |
| 1 µM | - | - | - | - | - | - | + | + | + | ++ | + | ++ |
| 0.5 µM | - | - | - | - | - | - | + | + | + | + | ++ | + |
| 0.1 µM | - | - | - | - | - | - | + | + | + | + | ++ | + |

**Lecture à 3 jours**

| | Témoins | | | 10 TCID50 | | | 100 TCID50 | | | 500 TCID50 | | |
|---|---|---|---|---|---|---|---|---|---|---|---|---|
| DABF-AmB '/HUT78/SIV | 1 | 2 | 3 | 4 | 5 | 6 | 7 | 8 | 9 | 10 | 11 | 12 |
| tem nég./pos. | - | - | - | + | + | + | + | + | + | +++ | +++ | +++ |
| 100 µM | - | - | - | - | cytotoxicité | | | - | - | - | - | - |
| 50 µM | - | - | - | - | - | - | - | - | - | - | - | - |
| 10 µM | - | - | - | - | - | - | - | - | + | - | + | + |
| 5 µM | - | - | - | - | - | + | - | - | + | + | + | - |
| 1 µM | - | - | - | + | + | + | + | ++ | + | +++ | ++ | +++ |
| 0.5 µM | - | - | - | + | + | + | + | + | ++ | ++ | +++ | ++ |
| 0.1 µM | - | - | - | + | + | + | ++ | + | + | + | +++ | +++ |

**Lecture à 5 jours**

EXEMPLE 8 - Inhibition par DABF-AmB de l'infection des PBMC par différents VIH-1 et par des virus résistants à l'AZT.

Des PBMC ont été infectés dans les conditions décrites aux protocoles généraux ci-dessus, par différents isolats de VIH-1 provenant de patients, ou identifiés comme résistants à l'AZT. L'effet anti-viral du DABF-AmB a été déterminé par la mesure de l'activité RT dans les surnageants après 10 jours de culture.

Comme le montre la figure 5, le DABF-AmB, à 10 $\mu$M est capable de diminuer considérablement, voire d'inhiber totalement l'infection de PBMC par 10 souches virales provenant de patients. La figure 6 montre que cette inhibition se manifeste également dans le cas de souches résistantes à l'AZT.

EXEMPLE 9 - Inhibition de la virémie cellulaire par DABF-AmB

Des lymphocytes de patient sidéens (<200CD4) sont isolés sur gradient de Ficoll à partir de sang hépariné. Les cellules sont reprises dans du milieu RPMI1640 contenant des antibiotiques, 10 U/ml d'IL2 recombinante (Boeringher) et du sérum anti-interféron alpha (anti-IFN$\alpha$, Sigma) plus 10% de sérum foetal de veau. Les cellules sont cultivées dans des plaques de 24 puits à raison de 5 x $10^5$ cellules par puits pour la première concentration puis successivement diluées de 5 en 5 dans le même milieu. Le milieu est renouvelé par moitié 2 fois par semaine. Chaque semaine, 3 x $10^5$ PBMC allogéniques fraîches, provenant de donneurs sains, sont ajoutées dans les cultures. Les cultures sont maintenues pendant 28 jours en présence de concentrations variables de DABF-AmB. La production virale est mesurée chaque semaine par le dosage de la protéine p24 dans les surnageants. Chaque culture et chaque dosage sont effectués en double.

Le tableau IV montre les résultats obtenus chez 8 patients différents. Les indications (-) signifient que la quantité de p24 détectée est inférieure d'au moins 95% aux valeurs obtenues dans les cultures témoin non traitées. Ces résultats montrent que la molécule DABF-AmB inhibe la virémie cellulaire chez 7 patients sur 8. Les résultats obtenus chez le patient LOU indiquent soit une résistance du virus au produit, soit une charge virale trop importante au départ. Ces expériences ont permis d'évaluer l'$IC_{50}$ du DABF-AmB entre 1 et 5 $\mu$M pour les différents isolats viraux ($IC_{50}$ = Concentration de produit qui provoque 50% d'inhibition du titre de p24 dans les surnageants de culture pour une dilution de cellules des patients correspondant à une charge virale égale à 10 $TCID_{50}$ ).

TABLEAU IV

EFFET DU DABF-AmB SUR LES VIREMIES CELLULAIRES

| DAY | RIF | RIF+MS | PHU | PHU+MS | LOU | LOU+MS | VUL | VUL+MS | MAI | MAI+MS | VAC | VAC+MS | CAM | CAM+MS | SAM | SAM+MS |
|---|---|---|---|---|---|---|---|---|---|---|---|---|---|---|---|---|
| | | | | | | | | | | | | | | | | |
| 0 | - | - | - | - | - | - | - | - | - | - | - | - | - | - | - | - |
| 7 | + | - | + | - | + | - | + | - | ++ | - | | | | | | |
| 14 | ++ | - | ++ | - | ++ | ++ | ++ | - | | | ++ | - | ++ | - | +/- | - |
| 21 | ++ | - | ++ | - | ++ | ++/- | ++ | - | | | | | | | | |
| 28 | ++ | - | ++ | - | ++ | ++/- | ++ | - | | | | | | | | |

EXEMPLE 10 - Action du DABF-AmB sur l'expression virale dans des cellules infectées réactivées.

L'activation des cellules CD4+ infectées est connue pour stimuler la production du virus, L'effet du DABF-AmB a donc été testé sur l'expression virale dans des cellules de patients sidéens réactivées in vitro par des anti-CD3 immobilisés, des anti CD28, la PMA (phorbol myristate acétate) ou des anti-CD3 solubles.

Des PBMC de patients (<300 CD4) ont été isolés sur gradient de Ficoll à partir du sang hépariné. Les cellules CD4 ont été isolées par tri cellulaire au moyen d'un cytomètre de flux (FACS-Star, Becton-Dickinson, Mountain View, CA) après marquage par OKT4-FITC. Les cellules CD4 ont été relavées puis remises en culture comme indiqué en 5 et activées soit par des anticorps anti-CD3, des anti-CD28 ou de la PMA. L'expression virale a été évaluée après 10 jours par le dosage de la p24 en ELISA dans les surnageants.

Les résultats présentés sur le tableau V montrent que chez 7 patients testés, DABF-AmB inhibe plus de 90 % de l'expression virale à une concentration de 10 μM. Ces résultats montrent que DABF-AmB est capable d'inhiber la production de virus lors de la réactivation de cellules préinfectées.

TABLEAU V

EFFET DU DABF AmB SUR LA PRODUCTION DE HIV DANS LES CELLULES $CD4^+$ PROVENANT DE PATIENTS INFECTES.

| PATIENTS | STIMULATION | | | | | | | | | |
|---|---|---|---|---|---|---|---|---|---|---|
| | cCD3 | cCD3 +MS | CD28 | CD28 +MS | cCD3 +CD28 | cCD3 +CD28 +MS | PMA | PMA +MS | sCD3 | sCD3 +MS |
| | | | | | | | | | | |
| S23 (PP) | | | | | | | + | - | | |
| S24 (I) | | | | | | | +/- | - | | |
| S25 (PP) | | | | | | | | | | |
| S26 (PP) | +/- | | | | ++ | - | | | | |
| S27 (PP) | | | | | | | | | | |
| S28 (PP) | | | | | | | | | | |
| S29 (I) | | | | | + | - | | | | |
| S30 (I) | +/- | - | ++ | | ++ | - | | | | |
| S31 (PP) | | | + | - | | | | | | |
| S32 (PP) | + | - | ++ | - | + | - | | | + | +/- |
| S33 (I) | | | | | | | | | | |
| S34 (PP) | | | | | | | | | | |
| S35 | | | | | | | | | | |
| S36 | | | | | | | | | | |

## II) - ACTION DU DABF-AmB SUR LE RHINOVIRUS HRV14

EXEMPLE 11 - Inhibition par DABF-AmB des effects cytopathogènes du HRV14

Des cellules HeLa Ohio cultivées jusqu'à confluence, sont infectées avec une suspension de virus HRV14. L'effet cytopathogène du virus est évalué en colorant les cellules avec une solution de 0,5% de violet cristal et 20% de méthanol ; après rincage des plaques à l'eau, on procède à la lecture de la densité optique à 550nm.

Pour la suite de l'expérimentation, les cellules sont infectées avec une suspension virale titrée de façon à provoquer 50% de mort cellulaire après 3 jours sur des cellules témoin ne recevant aucun traitement. 1 heure après l'infection, le produit à tester ( AmB ou DABF-AmB) est ajouté à des concentrations variant de 0,03 à 100 μM. Le pourcentage d'inhibition de l'effet cytopathogène est déterminé comme suit :

$$\frac{[\text{DO(cellules infectées traitées)} - \text{DO(cellules infectées]}}{[\text{DO (cellules saines)} - \text{DO (cellules infectées)]}}$$

Dans ces conditions, l'AmB n'a aucune activité antivirale détectable, et est cytotoxique aux concentrations supérieures à 5 μM. Au contraire, le DABF-AmB inhibe complètement les effets cytopathogènes du virus à partir de 10μM ($IC_{50}$ (50% d'inhibition de l'effet cytopathogène) = 5μM), et aucun effet cytotoxique du DABF-AmB n'a été décelé après un traitement des cellules par 20μM pendant 4 semaines. La N-méthyl glucamine et le benzylidène glucoside n'ont aucun effet sur le virus.

Lorsque la suspension de HRV14 est préincubée à 37°C en présence de DABF-AmB pendant 5 heures, on n'observe aucune inhibition de l'effet cytopathogène, par rapport à une suspension témoin incubée dans les mêmes conditions en l'abscence de DABF-AmB. Le DABF-AmB n'a donc pas d'effet virucide direct sur HRV14.

L'effet de l'addition de DAMBF-AmB a été testé à différents moments : préincubation des cellules avec le DABF-AmB avant l'infection virale ; addition du DABF-Amb au moment de l'infection ; addition du DABF-Amb après l'infection, pendant la période de prolifération virale. Les résultats obtenus montrent que le DABF-Amb agit essentiellement pendant la prolifération virale.

Ainsi que cela ressort de ce qui précède, l'Invention ne se limite nullement à ceux de ses modes de réalisation et d'application qui viennent d'être décrits de façon plus explicite ; elle en embrasse au contraire toutes les variantes qui peuvent venir à l'esprit du technicien en la matière, sans s'écarter du cadre, ni de la portée, de la présente Invention.

## Revendications

**1)** Utilisation de l'amphothéricine B, ou d'un dérivé de celle-ci, pour l'obtention d'un inhibiteur de protéases, actif en particulier sur des sérine-protéases, telles que la trypsine, la kallicréine, l'élastase leucocytaire.

**2)** Utilisation selon la revendication 1, caractérisé en ce que le dérivé d'amphothéricine B utilisé est choisi parmi les dérivés dont l'activité inhibitrice sur la trypsine est au moins deux fois supérieure à celle de l'amphothéricine B

**3)** Utilisation selon la revendication 2, caractérisée en ce que le dérivé d'amphothéricine B utilisé porte un substituant sur la fonction mycosamine.

**4)** Utilisation selon la revendication 3, caractérisée en ce que la fonction mycosamine de l'amphothéricine B est substituée par un groupe aspartyle.

**5)** Utilisation selon la revendication 3, caractérisée en ce que la fonction mycosamine de l'amphothéricine B est substituée par un groupe amino-1-désoxy-1-cétose, lui même éventuellement substitué.

**6)** Utilisation selon la revendication 5, caractérisée en ce que le dérivé utilisé répond à la formule générale (I).

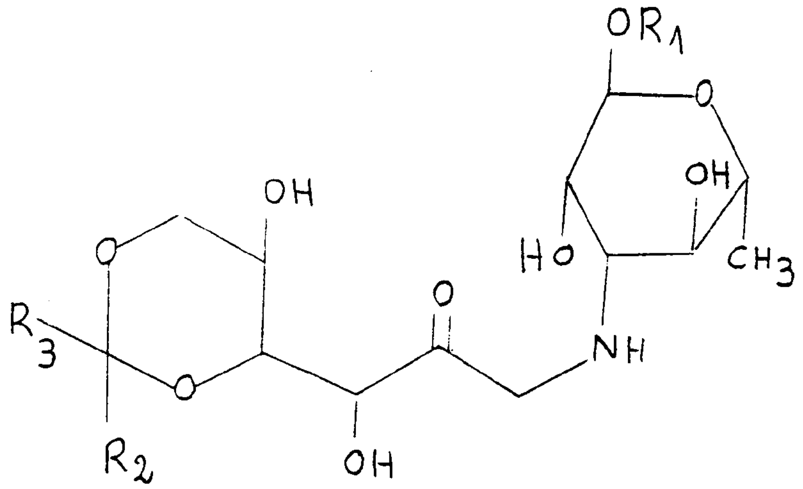

(I)

dans laquelle :

- R1 représente la partie macrocyclique d'un macrolide polyénique,
- R2 représente un atome d'hydrogène ou un groupe méthyle,
- et R3 représente un groupe méthyle, éthyle, propyle, phényle.

**7)** Utilisation selon la Revendication 6, caractérisée en ce que le dérivé utilisé est le 1-déoxy-1-amino-4,6-0-benzylidène-D-fructosyl-AmB.

**8)** Utilisation d'un inhibiteur de protéases tel que défini dans une quelconque des revendications 1 à 7 pour l'obtention d'un inhibiteur de la multiplication d'un agent pathogène dont le cycle de multiplication implique l'intervention d'au moins une enzyme protéolytique, du type sérine-protéase.

**9)** Utilisation selon la revendication 8, caractérisée en ce que ledit agent pathogène est un virus.

**10)** Utilisation selon la revendication 9, caractérisée en ce que ledit virus est le virus VIH-1.

**11)** Utilisation selon la revendication 9, caractérisée en ce que ledit virus est le virus VIH-2.

**12)** Utilisation selon la revendication 9, caractérisée en ce que ledit virus est le virus SIV.

**13)** Utilisation selon la revendication 9, caractérisée en ce que ledit virus est un rhinovirus.

**14)** Utilisation selon la revendication 8, caractérisée en ce que ledit agent pathogène est une bactérie.

**15)** Utilisation selon la revendication 8, caractérisée en ce que ledit agent pathogène est un organisme eucaryote, parasite de l'homme ou des animaux.

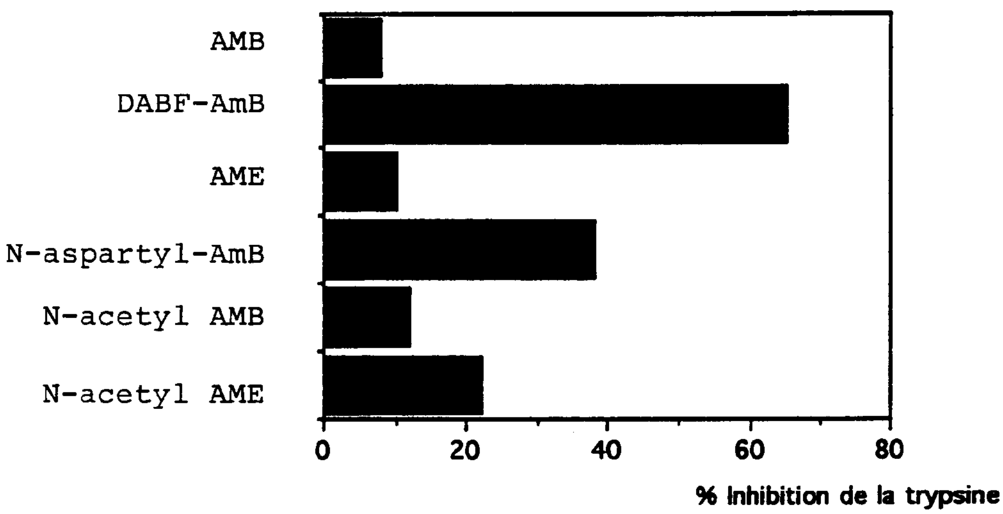
AMB
DABF-AmB
AME
N-aspartyl-AmB
N-acetyl AMB
N-acetyl AME
0
20
40
60
80
% Inhibition de la trypsine

FIGURE 1

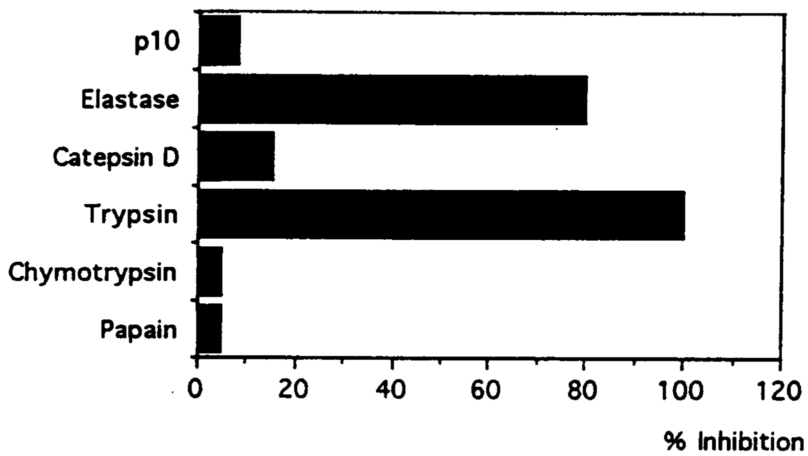
p10
Elastase
Catepsin D
Trypsin
Chymotrypsin
Papain
0
20
40
60
80
100
120
% Inhibition

FIGURE 2

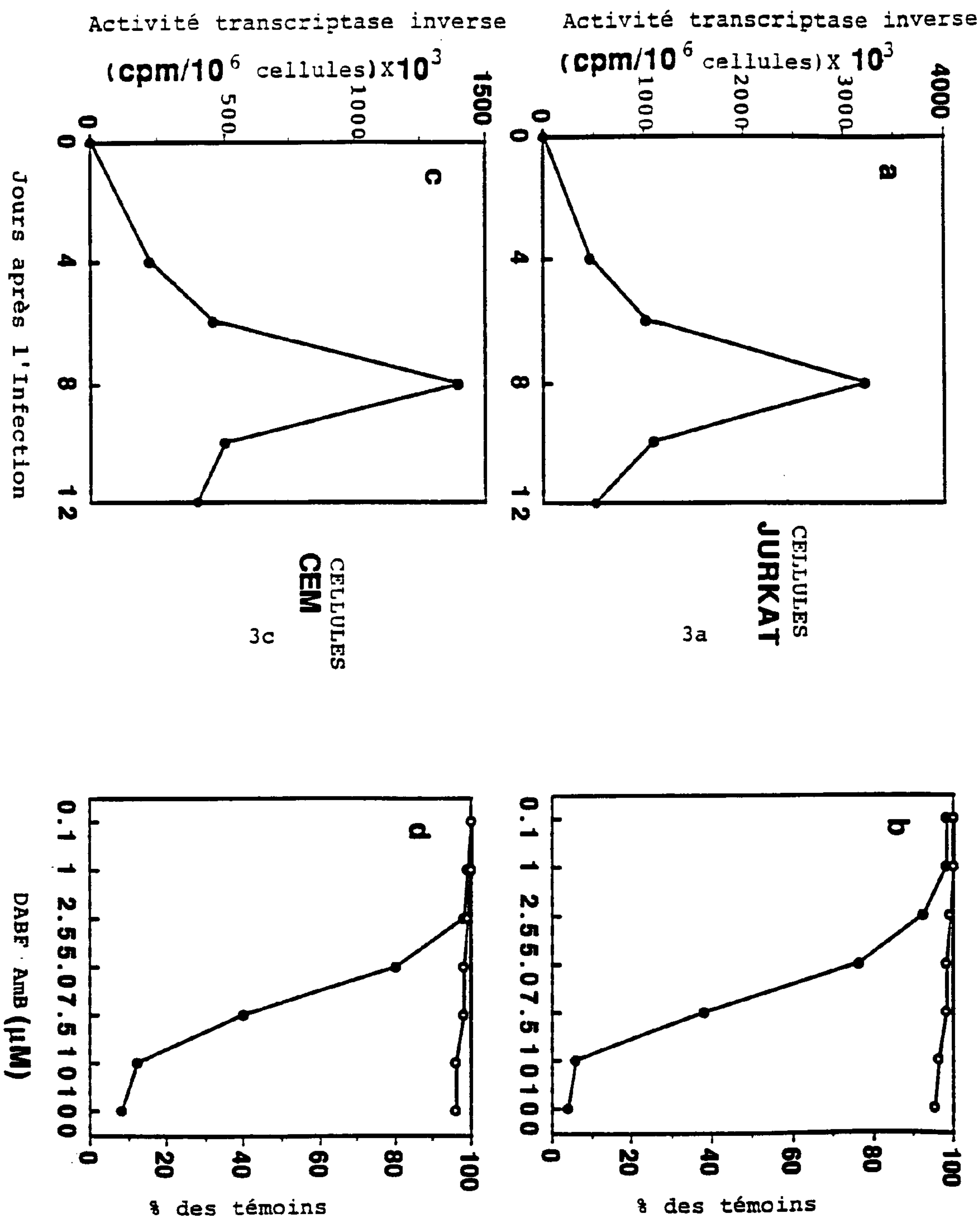


3c 3a

3d 3b

FIGURE 3

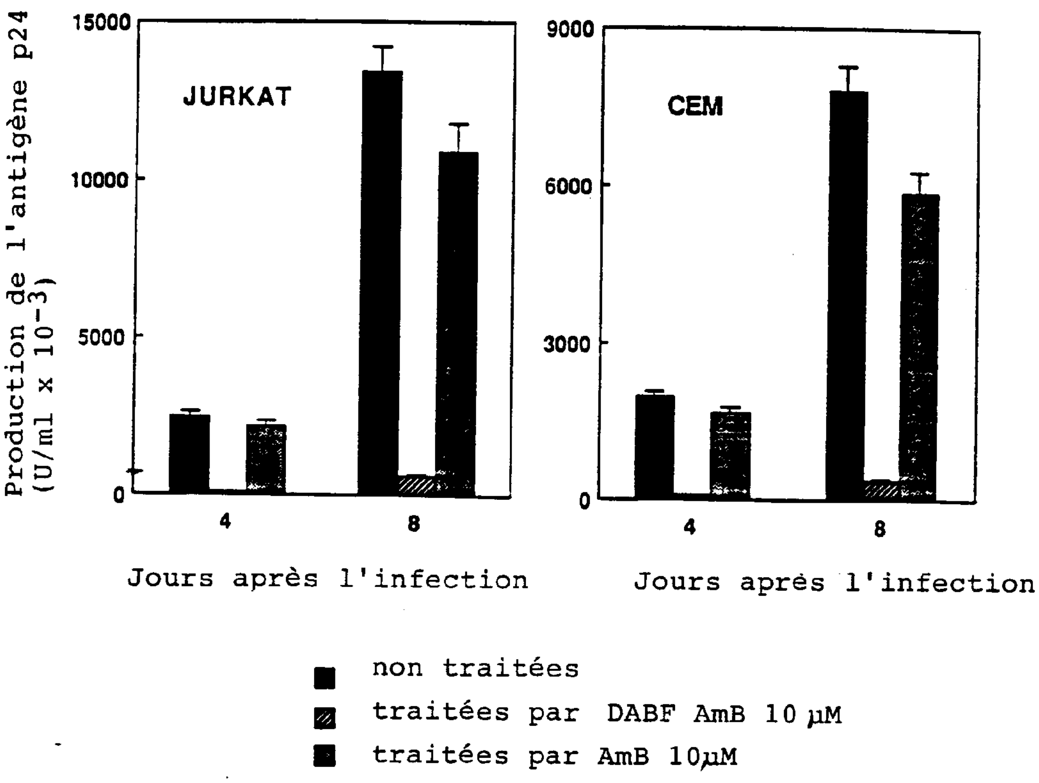
Production de l'antigène p24
(U/ml x 10-3)
15000
10000
5000
0
JURKAT
4
8
Jours après l'infection
9000
6000
3000
0
CEM
4
8
Jours après l'infection
non traitées
traitées par DABF AmB 10 µM
traitées par AmB 10µM

FIGURE 4

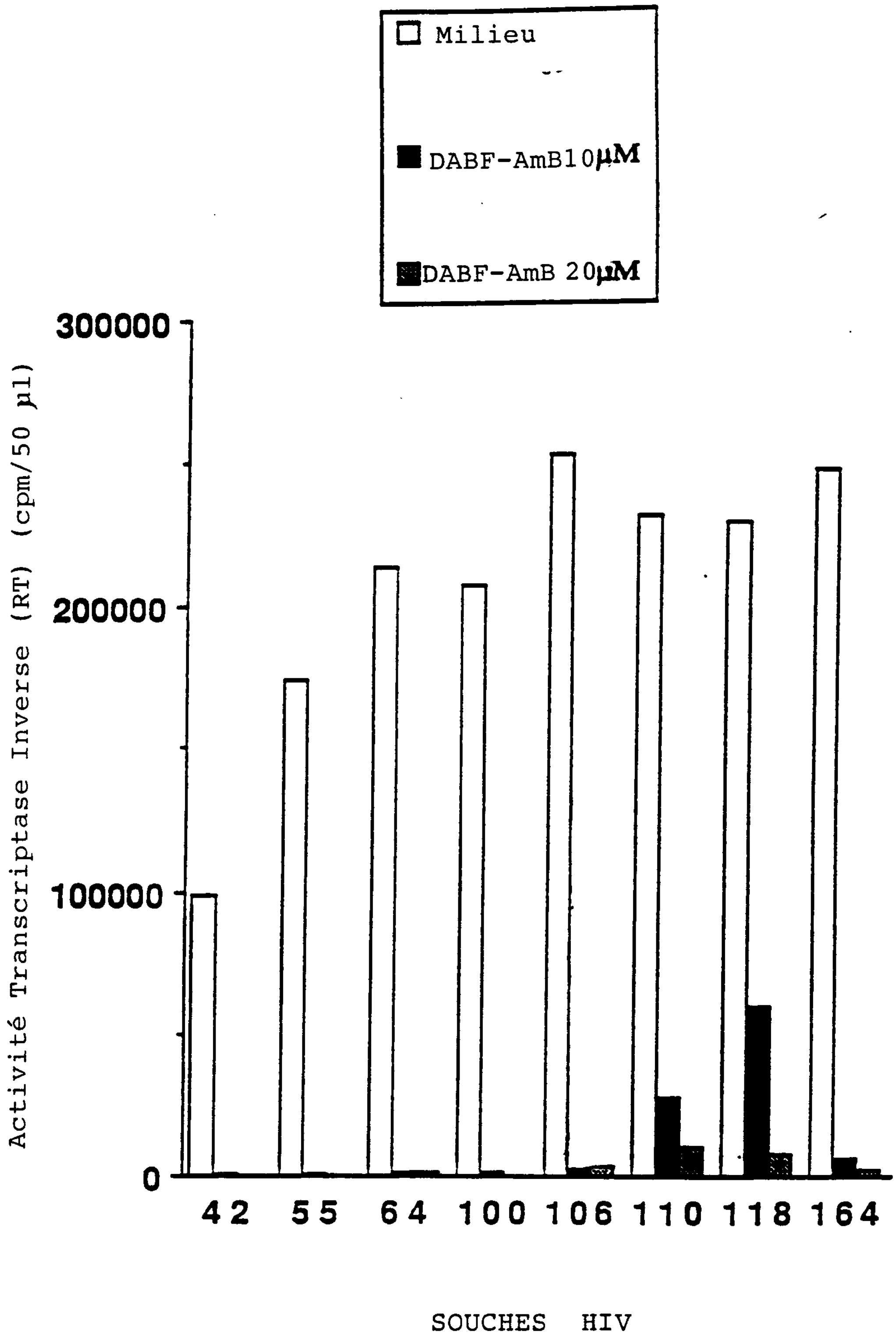
Milieu
DABF-AmB10μM
DABF-AmB 20μM
300000
200000
100000
0
Activité Transcriptase Inverse (RT) (cpm/50 μl)
42
55
64
100
106
110
118
164
SOUCHES HIV

FIGURE 5

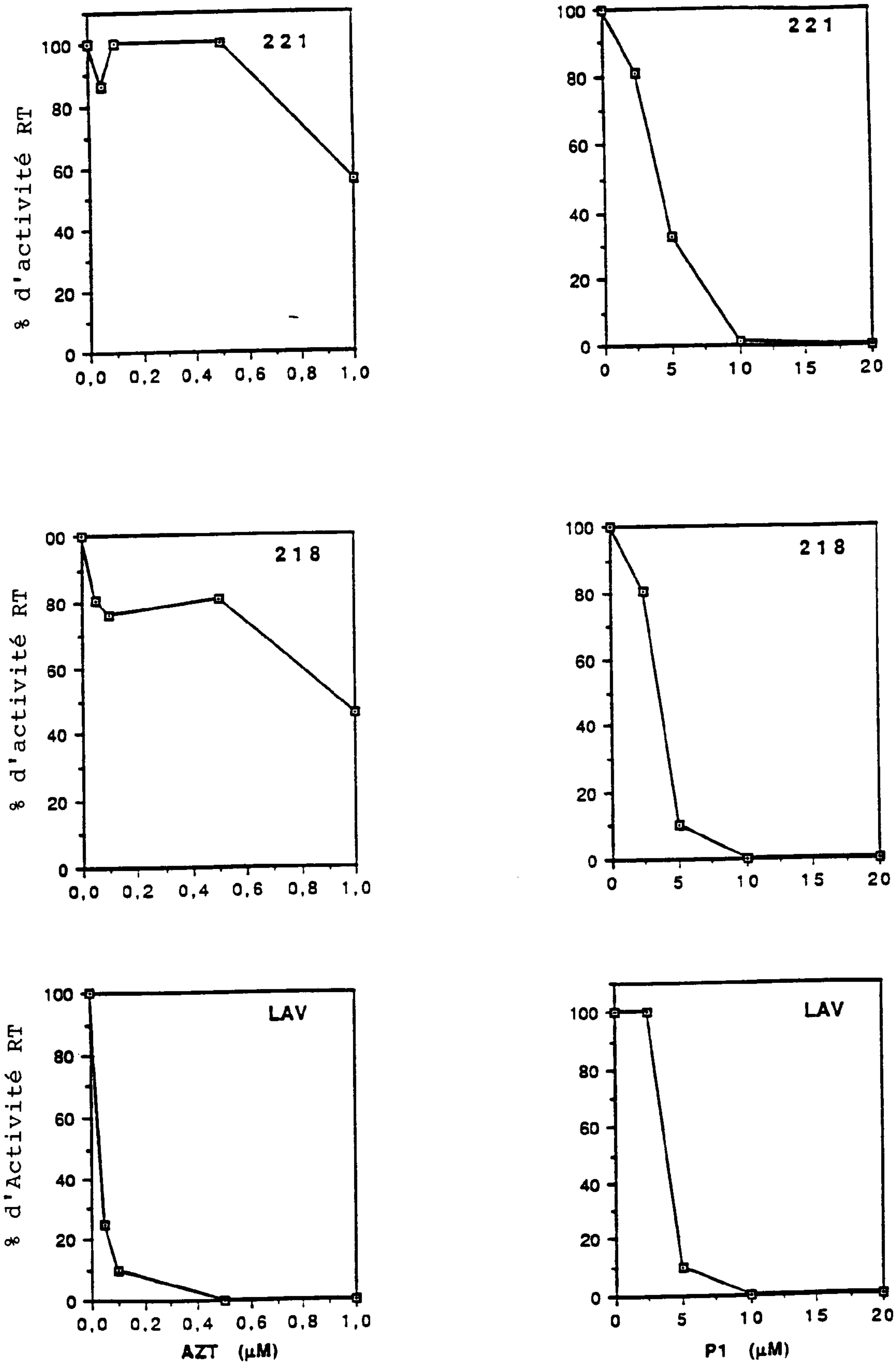
% d'activité RT
221
0,0 0,2 0,4 0,6 0,8 1,0
221
0 5 10 15 20
% d'activité RT
218
0,0 0,2 0,4 0,6 0,8 1,0
218
0 5 10 15 20
% d'Activité RT
LAV
0,0 0,2 0,4 0,6 0,8 1,0
AZT (µM)
LAV
0 5 10 15 20
P1 (µM)

FIGURE 6